# EUROPÄISCHE PATENTSCHRIFT

(11) **EP 0 342 253 B1**
(45) Veröffentlichungstag und Bekanntmachung des Hinweises auf die Patenterteilung: **13.01.1993**
(21) Anmeldenummer: 88107939.6
(22) Anmeldetag: 18.05.1988
(51) Int. Cl.: G01L 5/16, G01G 19/44

(54) **Kraftaufnehmer zum Einbau in Messplattformen**
Mounting of a force transducer in a measuring platform
Montage d'un capteur de force dans une plate-forme de mesure

(43) Veröffentlichungstag der Anmeldung: 23.11.1989
(73) Patentinhaber: Kistler Instrumente AG, CH-8408 Winterthur (CH)
(72) Erfinder: Wolfer, Peter, CH-8451 Kleinandelfingen (CH); Sonderegger, Hans Conrad, CH-8413 Neftenbach (CH)
(74) Vertreter: Schmidt, Horst, Dr.

(56) Entgegenhaltungen:
- DE-A- 3 019 751
- FR-A- 2 285 603
- FR-A- 2 325 916
- GB-A- 1 530 711
- US-A- 3 566 163
- US-A- 3 582 691
- SOVIET INVENTIONS ILLUSTRATED, Woche J50, 2. Februar 1983, Derwent Publications Ltd, London, GB; & SU-A-905 671 (MOSC RADIO ELTRN) 15-02-1982
- METALWORKING PRODUCTION, Band 104, Heft 1, 6. Januar 1960, Seite 12, London, GB; "Drilling forces dynamometer for small holes"

## Beschreibung

Die Erfindung betrifft eine Messplattform mit wenigstens einem elektromechanischen Kraftaufnehmer gemäss Patentanspruch 1.

Messplattformen werden insbesondere in der Zerspanungstechnik und Biomechanik zur Messung von Kräften in ein oder mehreren Richtungen, ggf. auch zur Messung von Drehmomenten, verwendet. Bei Zerspanungsmessungen kann dadurch beispielsweise der Zerspanungsvorgang dynamisch analysiert werden, wodurch die Zerspanungsparameter optimiert werden können. In der Biomechanik kann der Bewegungsablauf, z.B. beim Gehen, analysiert werden, was eine Diagnose von Defekten ermöglicht. Derartige Messplattformen sind z.B. in der DE-A-19 52 522, DE-A-33 13 960 und CH-A-595 623 beschrieben.

Die in der Biomechanik oder Zerspanungstechnik verwendeten Messplattformen bestehen zumeist aus einer Deckplatte und einer Grundplatte oder einem Grundrahmen mit einem oder mehreren dazwischen angeordneten Kraftaufnehmern. Eine derartige Messplattform ist in der FR-A-2 325 916 beschrieben. Bei der bekannten Messplattform weist der Kraftaufnehmer einen stirnflächenseitigen mit Schraubenlöchern versehenen Flansch auf, der bündig auf der Grundplatte der Messplattform aufsitzt. Die Verbindung des Kraftaufnehmers mit der Grundplatte erfolgt über durch die Schraubenlöcher des Flansches durchgeführte, in Gewindebohrungen der Grundplatte eingeschraubte Befestigungsschrauben. Die Kraftaufnehmer der bekannten Anordnung sind daher nur unvollkommen oder gar nicht gegen äussere Umwelteinflüsse geschützt bzw. abgedichtet. Eine weitere Folge des bekannten Aufbaues der Messplattform sind eine grössere Bauhöhe sowie Einbusse an Steifigkeit des Messystems.

Der Erfindung liegt demgegenüber die Aufgabe zugrunde, eine Messplattform der eingangs erwähnten Art zu schaffen, bei der die vorerwähnten Nachteile im wesentlichen beseitigt sind. Insbesondere soll die Erfindung eine dichte, gegen äussere Einflüsse im wesentlichen abgeschirmte Montage der Kraftaufnehmer bei derartigen Messplattformen ermöglichen.

Diese Aufgabe wird erfindungsgemäss durch die Merkmale des Patentanspruches 1 gelöst. Danach ist der Montageflansch, der im Bereich wenigstens eines axialen Endes des Kraftaufnehmers vorgesehen ist, in einer entsprechend ausgebildeten Montagebohrung in der Grundplatte der Messplattform aufgenommen und damit durch Schweissen, Löten oder Kitten, permanent dicht verbunden. Die unterschiedlich bemessenen axialen Endbereiche des Kraftaufnehmers erlauben es den Aufnehmer von aussen durch die Montagebohrung einzuschieben, bis der Montageflansch in der Montagebohrung zu liegen kommt. Der Kraftaufnehmer wird nach der permanenten Verbindung mit der Montagebohrung integriertes, gegen äussere Einflüsse im wesentlichen abgeschirmtes Bestandteil der Grundplatte. Ferner hat der Kraftaufnehmer radiale Signalleitungen an einer zwischenliegenden Stelle zwischen seinen axialen Enden, die innerhalb der Grundplatte in geeigneten darin vorgesehenen Ausnehmungen zu liegen kommen und dadurch ebenfalls gegen äussere Einflüsse abgeschirmt sind. Im übrigen wird bezüglich anderer Weiterbildungen der Erfindung auf die Patentansprüche verwiesen. Zwar ist aus der FR-A-2 285 603 eine Anordnung mit einem Kraftaufnehmer bekannt, der in weitgehender Übereinstimmung mit dem Merkmal a) des Patentanspruches 1 einen radialen Montageflansch aufweist, über den sich der Kraftaufnehmer auf einer Absatzfläche einer Schulterbohrung in einem Bauteil abstützen kann. Die bekannte Anordnung ist jedoch für die Messung der Drücke in Flüssigkeiten und Gasen ausgelegt, so dass der Kraftaufnehmer diesen Umwelteinflüssen ausgesetzt ist. Ausserdem kann bei der bekannten Anordnung eine Signalableitung nur durch den Aufnehmer selbst und den zugehörigen Montagehilfsmitteln erfolgen.

Die Erfindung wird nachfolgend anhand von Ausführungsformen und der Zeichnung näher erläutert. Es zeigen:
- Fig. 1: einen Kraftaufnehmer und eine Messplattform bekannter Konstruktion,
- Fig. 2: in fragmentarischer längsgeschnittener Ansicht einen Kraftaufnehmer in einer Messplattform gemäss einer Ausführungsform der Erfindung,
- Fig. 3: die Anordnung nach Fig. 2 in geschnittener Seitenansicht mit weggelassener Deckplatte,
- Fig. 4: die Anordnung nach Fig. 2 in Draufsicht mit weggelassener Deckplatte,
- Fig. 5: in fragmentarischer perspektivischer geschnittener Ansicht einen Kraftaufnehmer in einer Messplattform gemäss einer weiteren Ausführungsform der Erfindung,
- Fig. 6: die Anordnung nach Fig. 5 in Draufsicht,
- Fig. 7 und 8: Ansichten zur Erläuterung der Montage von Kraftaufnehmer und Grundplatte zur Schaffung der Messplattformen nach Fig. 2 bzw. 5,
- Fig. 9: in fragmentarischer geschnittener perspektivischer Ansicht einen Kraftaufnehmer in einer Messplattform gemäss einer weiteren Ausführungsform der Erfindung,
- Fig. 10: in fragmentarischer geschnittener perspektivischer Ansicht einen Kraftaufnehmer in einer Messplattform gemäss einer weiteren Ausführungsform der Erfindung, und
- Fig. 11: in fragmentarischer geschnittener perspektivischer Ansicht einen Kraftaufnehmer in einer Messplattform gemäss einer weiteren Ausführungsform der Erfindung, angeordnet auf einer Bodenplatte.

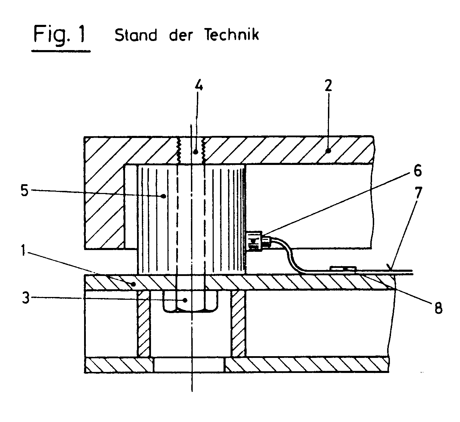

Fig. 1 stellt einen partiellen Längsschnitt durch eine konventionelle Messplattform dar, wie sie beispielsweise in der Biomechanik verwendet wird (Stand der Technik). Sie setzt sich zusammen aus einer Grundplatte 1, einer Deckplatte 2 und einem dazwischen liegenden Kraftaufnehmer 5, der seine Vorspannung über eine Vorspannschraube 3 erhällt, die in ein Gewinde 4 in der Deckplatte eingeschraubt ist. Der Kraftaufnehmer ist zentral durchbohrt, um die Vorspannschraube 3 aufzunehmen, ebenso der obere Teil der hier kastenförmig dargestellten Grundplatte 1. Das Festziehen der Vorspannschraube 3 kann mittels eines Schraubenschlüssels geschehen, welcher durch eine in der Figur 1 dargestellte, aber nicht bezeichnete Bohrung im unteren Teil der Grundplatte 1 durchgeführt werden kann. Da der Aufnehmer 5 selbst in Ansicht dargestellt ist, ist das beispielsweise aus piezoelektrischen Scheiben bestehende Messpaket nicht sichtbar. Die Uebermittlung der Signale erfolgt über einen Stecker 6 und eine Signalleitung 7, die mittels eines Befestigungselementes 8 beispielsweise an der Grundplatte 1 fixiert ist. Fig. 1 zeigt, dass die Messplattform nach dem Stand der Technik nicht dicht verschlossen ist. Verschmutzte, aggressive Flüssigkeiten und Gase können bis zu den empfindlichen Aufnehmern, Steckern und Signalleitungen vordringen. Überdies hat die Messplattform erhebliche Audehnung in Dickenrichtung.

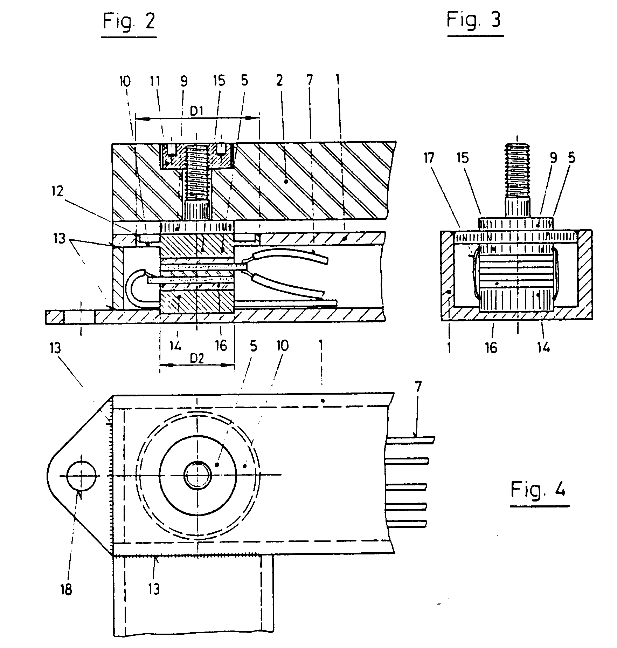

Fig. 2 stellt in einem partiellen Längsschnitt einen Kraftaufnehmer 5 dar, eingebaut einerseits in eine kastenförmige Grundplatte 1, andererseits in die krafteinleitende Deckplatte 2. Die Ziffern haben dieselbe Bedeutung wie in Fig. 1. Der Kraftaufnehmer 5 wird hier in seinen einzelnen Elementen sichtbar. So bedeutet 14 den Frontteil des Aufnehmers, welcher von einer nicht durchgehenden Bohrung im unteren Teil der Grundplatte 1 aufgenommen wird, 15 den Basisteil, der einen Flansch 10 trägt. Dazwischen liegt ein konventionell aufgebautes piezoelektrisches Messpaket 16 für Kraftmessung mit seitlichen Anschlüssen für Signalleitungen 7. Der zylinderförmige Krafteinleitungsteil 9 des Kraftaufnehmers 5 verjüngt sich zu einem nicht bezeichneten Fortsatz mit schraubenförmigem Endteil, der durch eine Bohrung in der Deckplatte 2 aufgenommen wird. Eine Spannmutter 11 trägt das entsprechende Innengewinde und liegt auf einem Absatz in der durchbohrten Deckplatte 1 auf. Durch Anziehen der Schraubenmutter 11 wird die Deckplatte 2 gegen den Krafteinleitungsteil 9 des Kraftaufnehmers 5 gepresst, wodurch die Messplattform zusammengehalten wird und die von aussen auf sie einwirkenden Kräfte auf das Messpaket 16 übertragen werden können.

Der Grundgedanke der Erfindung wird bei Fig. 2 sehr deutlich. Der Kraftaufnehmer 5 ist, samt den seitlichen Signalleitungen 7, in der kastenförmigen Grundplatte 1 voll integriert. Der frontseitige Montageflansch 10 ist mit der Grundplatte fest verbunden, z.B. durch Schweissen, Löten oder Kitten. Ebenso sind die Verbindungen 13 des Kastens gegen aussen dicht. Dadurch wird der Kasten völlig abgedichtet gegen äussere Einflüsse, welche sich auf die empfindlichen Kraftaufnehmer 5 schädlich auswirken könnten (Verschmutzung, Korrosion). Um diese Abdichtung sowie die Integration des Aufnehmers 5 in die Grundplatte 1 zu erreichen, muss dieser als fertiges Bauteil, samt den seitlichen Signalleitungen 7, in die kastenförmige Grundplatte 1 eingeschoben und nach diesem Vorgang fixiert werden können. Erfindungsgemäss geschieht dies auf einfache Weise derart, dass der Basisteil 15 des Kraftaufnehmers 5 mit einem Montageflansch 10 versehen ist, dessen Durchmesser grösser ist als der Durchmesser des Frontteils 14 des Aufnehmers 5. Der Flansch 10 passt in eine entsprechende Bohrung im oberen Teil der Grundplatte 1. Infolge der oben angegebenen Durchmesserverhältnisse vom Montageflansch 10 und der entsprechenden Bohrung sowie vom Frontteil 14 ist es nun einfach, den Aufnehmer 5 samt den Signalleitungen 7 in die Bohrung einzuschieben (die Signalleitungen 7 müssen vorzugsweise zuerst hindurchgeschoben werden). Bei gleich grossen Durchmessern von Montageflansch 10 und entsprechender Bohrung sowie Frontteil 14 wäre dies unmöglich. Nach dem Einschieben des Aufnehmers 5 erfolgt die Verschweissung des Montageflansches 10.

Fig. 3 und 4 zeigen dieselbe Messplattform wie Fig. 2, Fig. 3 zeigt sie im Querschnitt, Fig. 4 im Grundriss. Die Ziffern haben im folgenden immer dieselbe Bedeutung wie bis anhin. In Fig. 3 wird noch die elektrische Verbindung 17 zwischen Frontteil 14 und Basisteil 15 des Kraftaufnehmers 5 sichtbar (beide müssen sich auf demselben Potential, z.B. Erdpotential, befinden). Fig. 4 zeigt die rahmen- und kastenförmige Schweisskonstruktion der dargestellten Grundplatte 1. Die Bohrung 18 dient der Befestigung der Messplattform beispielsweise am Boden.

Die bisherigen Figuren stellen Messplattformen dar, wie sie vorzugsweise in der Biotechnik verwendet werden.

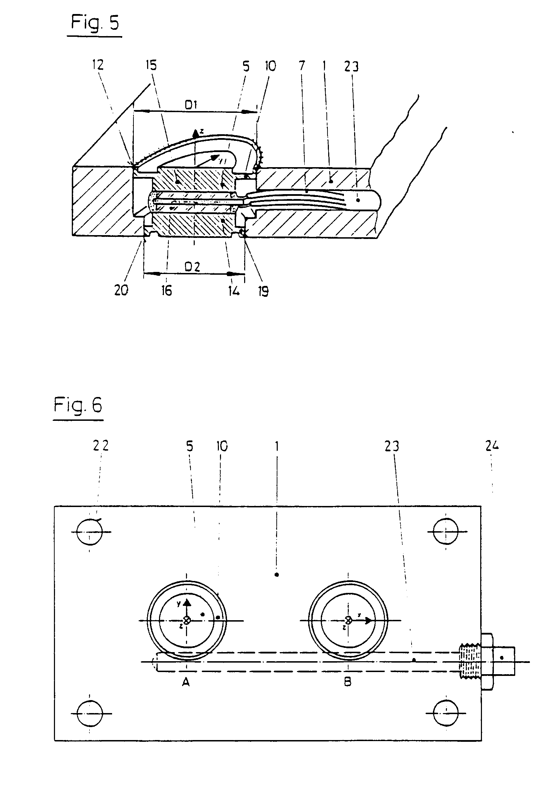

Fig. 5 zeigt einen Ausschnitt aus einer Messplattform, wie sie beispielsweise bei der Messung von Kräften und Drehmomenten bei Zerspanungsvorgängen Anwendung findet. Der Kraftaufnehmer 5 ist hier in eine volle Grundplatte 1 integriert. Lediglich für die Aufnahme der Signalleitungen 7 ist eine Ausnehmung 23 in der Grundplatte 1 angebracht. Der Aufnehmer 5 enthält ein mehrteiliges, konventionelles, piezoelektrisches Messpaket 16, das sowohl in der z-Richtung druck- als auch in der y- und/oder x-Richtung schubempfindlich ist. Bei einer vollen Grundplatte 1 liegt der Aufnehmer 5 vorzugsweise in einer nicht bezeichneten zweistufigen Durchgangsbohrung und trägt im Bereich beider axialer Enden Montageflansche 10, 19. Aus der Figur ist deutlich ersichtlich, dass der basisseitige Montageflansch 10 einen grösseren Durchmesser D₁ besitzt als der frontseitige Montageflansch 19 mit dem Durchmesser D₂. Dasselbe gilt für die entsprechenden dazu passenden Bohrungsdurchmesser in der Grundplatte 1. Bei der Montage können der fertige Aufnehmer 5 sowie die Signalleitungen 7 als Folge dieser Konstruktionsverhältnisse auf einfache Weise in die Durchgangsbohrung der Grundplatte 1 eingeführt werden, worauf die beiden Montageflansche 10, 19 eingeschweisst, eingelötet oder eingekittet werden können (dargestellt als Schweissungen 12, 20).

Die in Fig. 5 dargestellte Grundplatte 1 kann als einzelne Platte für Kraftmessungen beispielsweise in der Zerspanungstechnik Anwendung finden. Sie kann in eine passende Aussparung eines einzelnen Maschinenteils, aber auch zwischen zwei Maschinenteilen eingeschoben werden. Die für Kraftmessungen notwendige mechanische Vorspannung der Kraftaufnehmer 5 kann beispielsweise derart erzeugt werden, dass diese ein- oder beidseitig über die nicht bezeichneten Oberflächen der Grundplatte 1 um einen bestimmten, geringen Betrag herausragen. Im genannten Anwendungsbeispiel würden dann die Anschlussflächen der Aussparung gegen die Kraftaufnehmer 5 drücken (ev. unter Zwischenschaltung z.B. einer Folie) und diese vorspannen.

Fig. 6 zeigt den Grundriss einer vollständigen Grundplatte 1. Für die Messung eines Zerspanungsvorganges beispielsweise können ein oder mehrere Kraftaufnehmer 5 eingesetzt werden. Dargestellt sind hier zwei Kraftaufnehmer A und B, wovon beide in der z-Richtung druckempfindlich sind, und der eine (A) in der y-, der andere (B) in der x-Richtung schubempfindlich ist. Es können aber auch beide in derselben Richtung empfindlich sein, oder es können beide sowohl in der x- wie der y-Richtung schubempfindlich sein. Dargestellt ist ferner die Ausnehmung 23 für die Signalleitungen 7 (in dieser Figur nicht dargestellt) und die dichte Kabelanschlussbuchse 24 für den Anschluss externer Kabel, beispielsweise Koaxialkabel. Mit den bereits in Fig. 5 dargestellten dichten Einschweissungen der Kraftaufnehmer 5 bildet die Grundplatte 1 eine dichte, kompakte Messeinheit mit integrierten Kraftaufnehmern 5, die auch eine sehr hohe Steifigkeit und damit eine hohe Grenzfrequenz aufweist, und sich damit auch für die Analyse sehr rascher Vorgänge eignet, wie sie z.B. bei Zerspanungsvorgängen auftreten.

Die dargestellte Grundplatte 1 kann als solche beispielsweise zwischen zwei Maschinenteilen (oder, bei biomechanischen Anwendungen, am Boden) angebracht und mit einem Teil (oder dem Boden) fest verschraubt werden, mit Befestigungsschrauben durch die Bohrungen 22. Um eine definierte und sichere Krafteinleitung zu erreichen, kann die Grundplatte 1 auch zwischen zwei Krafteinleitungsplatten (hier nicht dargestellt) liegen und mit ihnen eine Messplattform bilden. Die notwendige mechanische Vorspannung der Kraftaufnehmer kann derart erreicht werden, dass deren Höhe so bemessen ist, dass sie auf einer oder beiden Seiten der Grundplatte 1 um einen bestimmten Betrag hervorragen. Durch Zusammenpressen der Krafteinleitungsplatten, beispielsweise mit vier Vorspannschrauben durch die Bohrungen 22 lässt sich die angemessene Vorspannung erreichen.

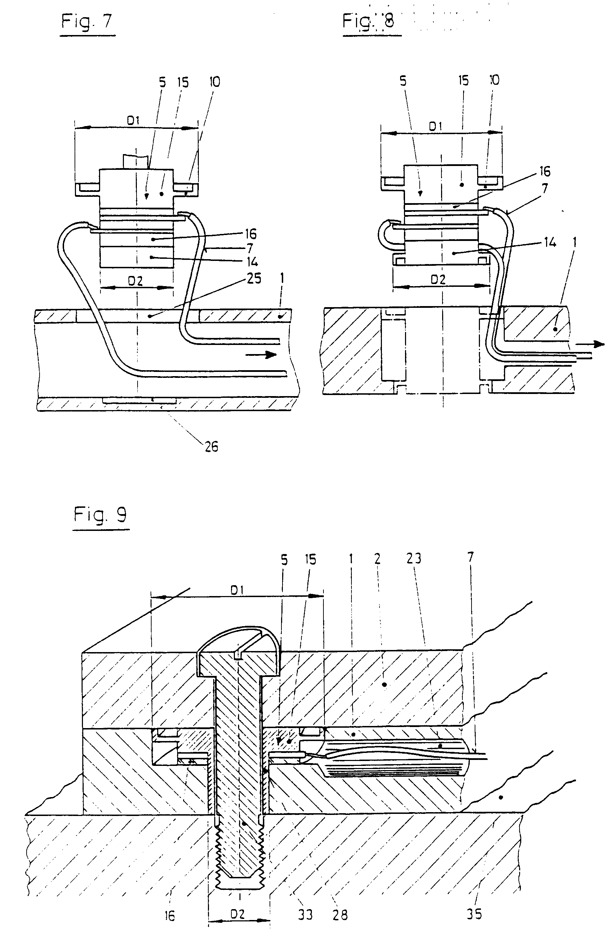

Fig. 7 und 8 stellen, zusammenfassend, nochmals die wichtigsten erfindungsgemässen Einbauarten der Kraftaufnehmer dar. Bei Fig. 7 besteht die Grundplatte 1 aus einem Kasten, ist also hohl, bei Fig. 8 ist sie, bis auf Ausnehmungen, voll. Der Kraftaufnehmer 5 bei der Fig. 7 trägt nur an seinem basisseitigen Ende ("oben" in der Figur) einen Montageflansch 10 mit dem Durchmesser D₁, das entgegengesetzte, frontseitige Ende mit dem Durchmesser D₂ trägt keinen. Der basisseitige Durchmesser D₁ ist grösser als der frontseitige D₂. Der Montageflansch 10 passt in die Montagebohrung 25 der Grundplatte 1, der Frontteil 14 des Aufnehmers 5 passt in die nicht durchgehende Aufnahmebohrung 26. Bei der Montage des fertigen Aufnehmers 5 in die kastenförmige Grundplatte 1 werden vorteilhafterweise zuerst die Signalleitungen 7 ein- und seitlich weggeführt, worauf der Aufnehmer 5 nachgeschoben werden kann. Die im wesentlichen volle Grundplatte 1 in Fig. 8 (entsprechend der Grundplatte in Fig. 5) enthält eine zweistufige Durchgangsbohrung für den mit zwei Montageflanschen 10, 19 versehenen Kraftaufnehmer 5, wobei der Durchmesser D₁ des basisseitigen Montageflansches 10 wiederum grösser ist als der Durchmesser D₂ des frontseitigen Montageflansches 19. Diesen beiden Durchmessern entsprechen die Durchmesser der zweistufigen Durchgangsbohrung. Auch hier werden, wie dargestellt, bei der Montage des Aufnehmers 5 zuerst die Signalleitungen 7 in die basisseitige Bohrung in der Grundplatte 1 eingeführt und dann der Aufnehmer 5 in die Durchgangsbohrung eingeschoben, bis er die in Fig. 8 punktiert eingezeichnete Position einnimmt.

Fig. 7 und Fig. 8 machen deutlich, dass die Montage des Kraftaufnehmers in die Grundplatte dadurch wesentlich vereinfacht wird, dass der Aufnehmer an seinem basisseitigen Ende ("oben" in der Figur) einen grösseren Durchmesser besitzt als an seinem frontseitigen Ende, und dass die Dichtheit des Messystems im Falle einer kastenförmigen Grundplatte das Einschweissen eines Montageflansches bedingt, während bei einer vollen Grundplatte im allgemeinen zwei Montageflansche eingeschweisst werden.

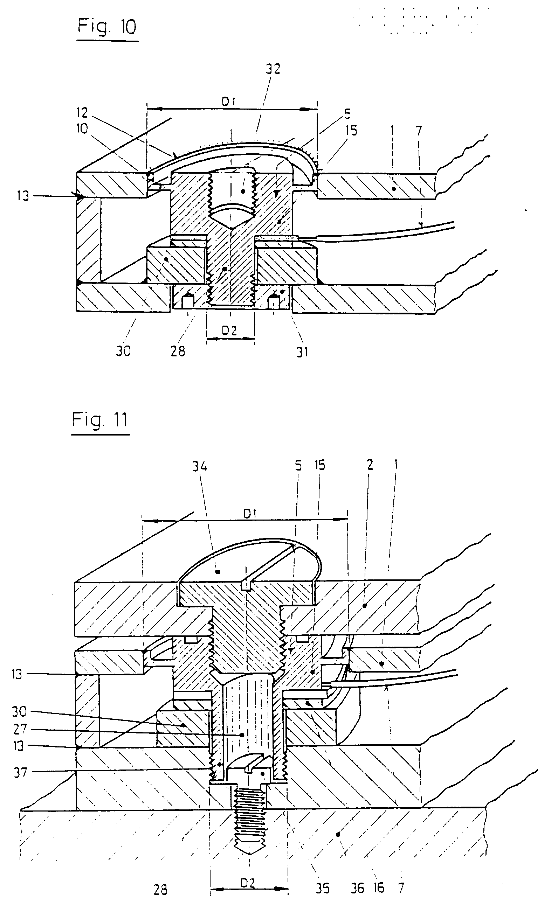

Die Figuren 9 und 10 stellen erfindungsgemässe Ausführungsformen von Messplattformen mit Kraftaufnehmern dar.

Fig. 9 stellt eine Messplattform dar, bestehend aus einer Grundplatte 1 und einer Deckplatte 2, welche mittels einer oder mehreren Befestigungsschrauben 33 beispielsweise an der Bodenplatte 36 verankert ist (biomechanische Anwendung). Die Messplattform kann aber auch zwischen zwei Maschinenteilen oder in einer Aussparung eines Maschinenteils angebracht sein. Die Grundplatte 1 entspricht im wesentlichen der in Fig. 5 gezeigten. Der Hauptunterschied besteht darin, dass der Kraftaufnehmer 5 eine zentrale Bohrung 27 besitzt. Ausserdem ist die Grundplatte 1, obschon sie im wesentlichen eine volle Platte darstellt, mit einer zweistufigen Durchgangsbohrung versehen, in welcher der mit nur einem Montageflansch 10 versehene Aufnehmer 5 seinen Platz findet. Das ebenfalls zentral durchbohrte piezoelektrische Messpaket 16 liegt zwischen dem Basisteil 15 des Aufnehmers 5 und der nicht bezeichneten Stufe der Durchgangsbohrung in der Grundplatte 1. Das Einschieben des Aufnehmers 5 in die Grundplatte 1 geschieht analog wie bei den in Fig. 5, 6 oder 7 dargestellten Ausführungsformen der Erfindung, was dadurch ermöglicht wird, dass auch hier der Aufnehmer 5 an seinem basisseitigen Ende einen grösseren Durchmesser (D₁) besitzt als an seinem frontseitigen Ende (D₂), das als rohrförmiger Fortsatz 28 in den engeren Abschnitt der zweistufigen Durchgangsbohrung passt. Die in Fig. 9 dargestellte Grundplatte 1 trägt eine Deckplatte 2 mit einer nicht bezeichneten Bohrung zwecks Aufnahme der durchgehenden Befestigungsschraube 33. Auch die Bodenplatte 36 enthält eine Bohrung mit Innengewinde (nicht bezeichnet) zwecks Aufnahme des Gewindeabschnittes der Befestigungsschraube 33. Wie bereits bei der Beschreibung von Fig. 5 dargelegt wurde, ist es im allgemeinen erforderlich, die Kraftaufnehmer 5 vor der Befestigung der Deckplatte 2 um einen gewissen Betrag über die Grundplatte 1 herausragen zu lassen, damit eine bestimmte mechanische Vorspannung durch Festziehen der Befestigungsschrauben 33, die gleichzeitig Vorspannschrauben sein können, überhaupt erzeugt werden kann. In dem in Fig. 9 dargestellten Fall wurde die Befestigungsschraube 33 so fest angezogen, dass die Deckplatte 2 die Grundplatte 1 kraftschlüssig berührt. Dadurch entsteht ein Kraftnebenschluss, d.h. der oder die Kraftaufnehmer 5 empfängt nicht mehr die volle einwirkende Kraft. Ein Teil davon wird direkt auf die Grundplatte 1 übertragen. Bei grossen Kräften kann dies ein Vorteil sein.

Fig. 10 zeigt die kastenförmige Grundplatte 1 für eine zweiteilige Messplattform, wie sie beispielsweise in der Biomechanik angewendet wird. Der Kraftaufnehmer 5 hat hier keine durchgehende zentrale Bohrung, weil die mechanische Vorspannung des piezoelektrischen Messpaketes 16 nicht mittels einer zentral durchgehenden, separaten Vorspannschraube erzeugt wird. Der Aufnehmer 5 besitzt an seinem frontseitigen Ende einen rohrförmigen Montagefortsatz 28, der mit einem Aussengewinde durch das nicht bezeichnete Innengewinde einer Spannmutter 31 aufgenommen wird. Diese Spannmutter 31 passt in eine Bohrung in einer Wand der kastenförmigen Grundplatte 1, auf deren Innenseite die ringförmige Unterlage 30 für das Messpaket 16 derart festgeschweisst ist, dass sie die genannte Bohrung überlappt. Durch Festziehen der Spannmutter 31 wird der Basisteil 15 des Kraftaufnehmers 5 gegen das Piezoelektrische Messpaket 16 gepresst, wodurch dieses mechanisch vorgespannt wird. Der Basisteil 15 des Kraftaufnehmers 5 enthält eine Bohrung 32 mit Gewinde zur Befestigung einer nicht eingezeichneten krafteinleitenden Deckplatte. Diese Deckplatte ist an den den Aufnehmern 5 gegenüberliegenden Stellen durchbohrt. Die Bohrungen nehmen die zum Gewinde der Bohrungen 32 passenden Befestigungsschrauben auf. Auch hier ist der basisseitige Durchmesser D₁ grösser als der frontseitige D₂, wodurch das Einschie ben der seitlich wegführenden Signalleitungen 7 möglich wird, worauf erst der Aufnehmer 5 eingeführt wird.

Fig. 11 stellt eine Weiterentwicklung der in den Figuren 9 und 10 dargestellten Messplattformen dar. Die in Fig. 10 dargestellte Grundplatte 1 trägt nun eine Deckplatte 2, welche mittels einer in die Gewindebohrung 32 der Kraftaufnehmer 5 (schon in Fig. 10 dargestellt) passende Befestigungsschraube 34 auf dem Kraftaufnehmer 5 festgeschraubt ist. Auch diese Messplattform kann einen oder mehrere Kraftaufnehmer enthalten. Der Basisteil 15 des Kraftaufnehmers 5 steht über die Grundplatte 1 vor. Dadurch kann erreicht werden, dass eine auf die Deckplatte 2 wirkende Kraft voll durch den oder die Kraftaufnehmer 5 aufgenommen wird, weil sich Grundplatte 1 und Deckplatte 2 nicht berühren (Krafthauptschluss). Der rohrförmige Fortsatz 28 des Kraftaufnehmers 5 trägt an seinem frontseitigen Ende ein Aussengewinde 37, welches in ein Innengewinde einer Bohrung (nicht bezeichnet) in der Grundplatte 1 passt. Durch Drehen des Kraftaufnehmers 5 beispielsweise mittels eines Schlüssels an seinem basisseitigen Ende kann das Messpaket 16 unter Vorspannung gesetzt werden. Die genannte Gewindebohrung in der Grundplatte 1 geht an ihrem frontseitigen Ende in eine glatte Bohrung geringeren Durchmessers über, wodurch eine Stufe entsteht, welche den Kopf einer Schraube 35 trägt. Die Schraube 35 kann durch ein entsprechendes Innengewinde einer Bohrung (nicht bezeichnet) in der Bodenplatte 36 aufgenommen werden. Durch Anziehen der Schraube 35, was vor der Befestigung der Deckplatte 2 geschehen muss, kann die Grundplatte 1 fest in der Bodenplatte 36 verankert werden. Diese Befestigungsart der Grundplatte 1 mit der Bodenplatte 36 kann durch mehrere Schrauben 35 vorgenommen werden, wenn die Messplattform mehrere Kraftaufnehmer 5 enthält. Auf diese Weise entsteht ein stabiles, fest verankertes Messystem. Die dargestellte Messplattform stellt eine bevorzugt in der Biomechanik verwendete Ausführungsform dar. Die Messplattform kann jedoch auch zwischen zwei Maschinenteilen liegen oder in einer Aussparung eines Maschinenteils. Die Bodenplatte 36 stellt dann eine Wandfläche eines Maschinenteils oder der genannten Aussparung dar.

Die vorliegende Erfindung bezieht sich auf Messplattformen oder Grundplatten, wie sie in der Zerspanungstechnik und Biomechanik angewandt werden. Sie ermöglicht auf einfache Weise die Integration von Kraftaufnehmern in die Grundplatte einer Messplattform oder in eine einzige Grundplatte. Bis anhin wurden die Kraftaufnehmer auf die Grundplatte montiert. Dadurch, dass die Kraftaufnehmer frontseitig einen kleineren Durchmesser besitzen als basisseitig, wobei die Aufnehmer in Bohrungen der Grundplatte passen, ist es möglich, die Kraftaufnehmer nach Kabelanschluss in die Aufnahmebohrungen zu versenken und einzuschweissen, einzulöten oder einzukitten. Dadurch entsteht eine universell anwendbare Grundplatte mit einem fest montierten Koaxial- oder Mehrfachstecker und vollkommen geschützten und dicht eingebrachten Kabelanschlüssen und Signalleitungen. Die Hohlräume können nach Einführung der Signalleitungen mit einem aushärtenden Gel ausgefüllt werden, damit diese besser isoliert und in ihrer Lage fixiert sind. Zudem können in die genannten Hohlräume Zwischenverstärker eingebracht werden. Die Steifigkeit der erfindungsgemässen Messplattformen ist wegen der kompakteren Bauform und der geringeren Dicke grösser als jene der bisherigen Messplattformen. Die in den Figuren dargestellten Ausführungsformen der Erfindung enthalten piezoelektrische Aufnehmerelemente. Es können aber auch andere elektromechanische Aufnehmerelemente wie z.B. piezoresistive oder mit Dehnungsstreifen vorgesehen werden. Die Messplattformen können einen oder mehrere Kraftaufnehmer enthalten, wobei die Kraftaufnehmer in einer oder beiden Richtungen (x, y) schubempfindlich, ev. zusätzlich in der z-Richtung druckempfindlich sein können. Es ist aber auch möglich, dass der oder die Kraftaufnehmer ausschliesslichin der z-Richtung druckempfindlich sind.

Die Herstellung der Messplattformen ist wegen des einfachen Einbaus der Kraftaufnehmer wesentlich rationeller als die Herstellung konventioneller Plattformen mit zwischenliegenden Kraftaufnehmern.

## Patentansprüche

1. Messplattform mit wenigstens einem elektromechanischen Kraftaufnehmer, folgende Merkmale umfassend:
a) der Kraftaufnehmer weist zur Befestigung an einer Grundplatte (1) der Meßplattform im Bereich seines einen axialen Endes einen im wesentlichen radialen Montageflansch (10) mit einem grösseren Aussendurchmesser als der Aussendurchmesser des Kraftaufnehmers im Bereich seines anderen axialen Endes auf,
b) der Montageflansch (10) des Kraftaufnehmers ist in einer Montagebohrung der Grundplatte (1) aufgenommen und damit in abdichtender Weise verschweisst, verlötet oder verkittet, und
c) vom Kraftaufnehmer gehen an einer zwischenliegenden Stelle zwischen seinen axialen Enden im wesentlichen radiale, innerhalb der Grundplatte (1) verlaufende Signalleitungen (7) ab.

2. Messplattform nach Anspruch 1, dadurch gekennzeichnet, dass ein weiterer im wesentlicher radialer, im Bereich des anderen axialen Endes des Kraftaufnehmers vorgesehener Montageflansch (19) mit kleinerem Aussendurchmesser (D2) als der Aussendurchmesser (D1) des einen Montageflansches (10) in einer zur einen Montagebohrung im wesentlichen axial ausgerichteten weiteren Montagebohrung der Grundplatte (1) aufnehmbar ist.

3. Messplattform nach Anspruch 1 oder 2, dadurch gekennzeichnet, dass die Grundplatte (1) als Kastenprofil ausgebildet ist.

4. Messplattform nach einem der Ansprüche 1 bis 3, dadurch gekennzeichnet, dass die Grundplatte (1) eine Vollplatte ist.

5. Messplattform nach einem der vorhergehenden Ansprüche, dadurch gekennzeichnet, dass der Kraftaufnehmer wenigstens einen von einem der axialen Enden sich erstreckenden axialen Montagefortsatz (28) aufweist.

6. Messplattform nach Anspruch 5, dadurch gekennzeichnet, dass der Montagefortsatz (28) in einer Zweistufenbohrung der Grundplatte (1) aufgenommen ist, und dass sich eine Befestigungsschraube (35) zur Befestigung der Grundplatte (1) an einem Untergrund auf einem Absatz der Zweistufenmontagebohrung abstützt.

7. Messplattform nach Anspruch 5, dadurch gekennzeichnet, dass der axiale Montagefortsatz (28) an dem dem einen Montageflansch (10) abgewandten axialen Ende des Kraftaufnehmers vorgesehen ist.

8. Messplattform nach Anspruch 5, dadurch gekennzeichnet, dass der Montagefortsatz (28) eine zentrale Durchgangsbohrung (27) zur Aufnahme einer Vorspannschraube (33) aufweist.

9. Messplattform nach Anspruch 5, dadurch gekennzeichnet, dass der Montagefortsatz (28) mit einem Gewinde zum Zusammenwirken mit einer an der Grundplatte (1) sich abstützenden Spannmutter (31) zum Vorspannen des Messpaketes des Kraftaufnehmers versehen ist.

10. Messplattform nach einem der vorhergehenden Ansprüche, dadurch gekennzeichnet, dass in dem dem einen Montageflansch (10) zugewandten axialen Ende des Kraftaufnehmers eine Gewindebohrung (32) eingebracht ist.

11. Messplattform nach einem der vorhergehenden Ansprüche, dadurch gekennzeichnet, die die Signalleitungen (7) des Kraftaufnehmers aufnehmenden Ausnehmungen der Grundplatte (1) mit einem isolierenden aushärtbaren Gel ausgegossen sind.

12. Messplattform nach einem der vorhergehenden Ansprüche, dadurch gekennzeichnet, dass in den Ausnehmungen der Grundplatte (1) ein oder mehrere Zwischenverstärker angeordnet sind.

## Claims

1. A measuring platform having at least one electromechanical force transducer, and comprising the following features:
a) for mounting the force transducer to a base plate (1) of the measuring platform the force transducer comprises on a portion near one axial end thereof a mounting flange (10), said mounting flange extends in an essentially radial direction and has a larger outer diameter than the outer diameter of the force transducer at a portion near the other axial end thereof,
b) the mounting flange (10) of the force transducer is received in a mounting hole of the base plate (1) and sealingly welded, soldered or cemented thereto, and
c) signal lines (7) extend inside the base plate (1) in an essentially radial direction from an intermediate portion of the force transducer between its axial ends.

2. The measuring platform according to claim 1, characterized in that another essentially radially extending mounting flange (19) having a smaller outer diameter (D2) than the outer diameter (D1) of the said one mounting flange (10) and provided on a portion near the other axial end of the force transducer is adapted to be received in another mounting hole of the base plate (1) essentially axially oriented with said one mounting hole.

3. The measuring platform according to claim 1 or 2, characterized in that the base plate (1) has a box-like cross section.

4. The measuring platform according to one of the claims 1 to 3, characterized in that the base plate (1) is a solid plate.

5. The measuring platform according to one of the preceding claims, characterized in that the force transducer comprises at least one mounting extension (28) axially extending from one of the axial ends of the force transducer.

6. The measuring platform according to claim 5, characterized in that the mounting extension (28) is received in a shoulder hole of the base plate (1), and that a mounting screw (35) for mounting the base plate (1) to a substrate abuts against a shoulder of the shoulder mounting hole.

7. The measuring platform according to claim 5, characterized in that the axial mounting extension (28) is provided at the axial end of the force transducer remote from the mounting flange (10).

8. The measuring platform according to claim 5, characterized in that the mounting extension (28) comprises a central through bore (27) for receiving a preloading screw (33).

9. The measuring platform according to claim 5, characterized in that the mounting extension (28) has a thread for cooperating with a clamp nut (31) for clamping together the measuring assembly of the force transducer, said clamp nut abuts against said base plate (1).

10. The measuring platform according to one of the preceding claims, characterized in that a thread hole (32) is provided in the axial end of the force transducer near the said one mounting flange (10).

11. The measuring platform according to one of the preceding claims, characterized in that an insulating setting gel is cast into the cavities of the base plate (1) receiving the signal lines (7) of the force transducer.

12. The measuring platform according to one of the preceding claims, characterized in that one or more booster amplifiers are disposed in the cavities of the base plate (1).

## Revendications

1. Plate-forme de mesure avec au moins un capteur de force électromécanique présentant les caractéristiques suivantes :
a) pour la fixation à une plaque de base (1) de la plate-forme de mesure le capteur de force présente, dans la zone de son extrémité axiale,un flasque de montage (10) essentiellement radial avec un diamètre extérieur plus grand que le diamètre extérieur du capteur de force dans la zone de son autre extrémité axiale,
b) le flasque de montage (10) du capteur de force est logé dans un trou de montage de la plaque de base (1) et est fixé à celle-ci de manière étanche par soudage, brasage ou mastiquage, et
c)des conducteurs de signaux (7) essentiellement radiaux, disposés dans la plaque de base (1), partent du capteur de force à un endroit intermédiaire entre ses extrémités axiales.

2. Plate-forme de mesure selon la revendication 1, caractérisée en ce qu'un autre flasque de montage (19) ayant un diamètre extérieur (D2) plus petit que le diamètre extérieur (D1) du premier flasque de montage (10) essentiellement radial et prévu dans la zone de l'autre extrémité axiale du capteur de force peut être logé dans un autre trou de montage de la plaque de base (1) qui est orienté en direction essentiellement axiale par rapport au premier trou de montage.

3. Plate-forme de mesure selon la revendication 1 ou 2, caractérisée en ce que la plaque de base (1) a un profil en caisson.

4. Plate-forme de mesure selon l'une des revendications 1 à 3, caractérisée en ce que la plaque de base (1) est une plaque pleine.

5. Plate-forme de mesure selon l'une des revendications précédentes, caractérisée en ce que le capteur de force présente au moins une embase de montage (28) axiale et s'étendant depuis l'une des extrémités axiales.

6. Plate-forme de mesure selon la revendication 5, caractérisée en ce que l'embase de montage (28) est reprise dans un trou à double gradin de la plaque de base (1) et qu'une vis de fixation (35),destinée à fixer la plaque de base (1) sur un support, prend appui sur un redan du trou de montage à double gradin.

7. Plate-forme de mesure selon la revendication 5, caractérisée en ce que l'embase de montage axiale (28) est prévue à l'extrémité axiale du capteur de force opposée au premier flasque de montage (10).

8. Plate-forme de mesure selon la revendication 5, caractérisée en ce que l'embase de montage (28) présente un trou de passage central (27) destiné à recevoir une vis de précontrainte (33).

9. Plate-forme de mesure selon la revendication 5, caractérisée en ce que l'embase de montage (28) est pourvue d'un filetage destiné à coopérer avec un écrou de serrage (31) prenant appui sur la plaque de base (1) afin de précontraindre le dispositif de mesure du capteur de force.

10. Plate-forme de mesure selon l'une des revendications précédentes, caractérisée en ce qu'un trou fileté (32) est pratiqué dans l'extrémité axiale du capteur de force tournée vers le premier flasque de montage (10).

11. Plate forme de mesure selon l'une des revendications précédentes, caractérisée en ce que les cavités de la plaque de base (1) recevant les conducteurs de signaux (7) du capteur de force sont renplies d'un gel isolant durcissable.

12. Plate-forme de mesure selon l'une des revendications prcédentes, caractérisée en ce qu'un ou plusieurs amplificateurs intermédiaires sont disposés dans les cavités de la plaque de base (1).
